# EUROPEAN PATENT SPECIFICATION

(11) **EP 3 140 381 B1**
(45) Date of publication and mention of the grant of the patent: **12.06.2019**
(21) Application number: 15722807.3
(22) Date of filing: 21.04.2015
(51) Int. Cl.: C10M 171/00, C07C 43/205, C10L 1/00, C10L 1/185

(54) **TETRARYLMETHANE ETHERS AS FUEL MARKERS**
TETRARYLMETHANETHER ALS BRENNSTOFFMARKER
ÉTHERS DE TÉTRARYLMETHANE COMME MARQUEURS DE CARBURANT

(30) Priority: 09.05.2014 US 201461990991 P
(43) Date of publication of application: 15.03.2017
(73) Proprietor: Rohm and Haas Company, Philadelphia, PA 19106 (US); Dow Global Technologies LLC, Midland, MI 48674 (US)
(72) Inventor: GREEN, George David, Cary, IL 60013 (US); SMITH, Warren E., Buckinghamshire Buckinghamshire HP9 1SE (GB); SWEDO, Raymond J., Mount Prospect, IL 60056 (US)
(74) Representative: Houghton, Mark Phillip
(86) International application number: PCT/US2015/026821
(87) International publication number: WO 2015/171305

(56) References cited:
- WO-A1-2012/177987
- WO-A1-2013/003573
- WO-A1-2013/165839
- WO-A1-2014/008164
- WO-A1-2014/165776

## Description

This invention relates to a new method for marking liquid hydrocarbons and other fuels and oils.

Marking of petroleum hydrocarbons and other fuels and oils with various kinds of chemical markers is well known in the art. A variety of compounds have been used for this purpose, as well as numerous techniques for detection of the markers, e.g., absorption spectroscopy and mass spectrometry. For example, WO2014/008164 discloses the use of trityl aryl ethers for use in marking liquid hydrocarbons and other fuels and oils. However, there is always a need for additional marker compounds for these products. Combinations of markers can be used as digital marking systems, with the ratios of amounts forming a code for the marked product. Additional compounds useful as fuel and lubricant markers would be desirable to maximize the available codes. The problem addressed by this invention is to find additional markers useful for marking liquid hydrocarbons and other fuels and oils.

### STATEMENT OF INVENTION

The present invention provides a method for marking a petroleum hydrocarbon or a liquid biologically derived fuel; said method comprising adding to said petroleum hydrocarbon or liquid biologically derived fuel at least one compound having formula C{Ph(R¹)ᵢ(OR²)ⱼ}₂{Ph(R³)ₘ(OR⁴)ₙ}{Ph(R⁵)ₒ(OR⁶)ₚ}, wherein Ph represents a benzene ring; R¹, R³ and R⁵ independently are C₁-C₁₈ alkyl or C₄-C₁₈ heteroalkyl; R², R⁴ and R⁶ independently are C₁-C₁₈ alkyl or C₄-C₁₈ heteroalkyl; i, m, n, o and p independently are zero, one or two; and j is one or two; wherein each compound having formula C{Ph(R¹)ᵢ(OR²)ⱼ}₂{Ph(R³)ₘ(OR⁴)ₙ}{Ph(R⁵)ₒ(OR⁶)ₚ} is present at a level from 0.01 ppm to 20 ppm.

### DETAILED DESCRIPTION

Percentages are weight percentages (wt%) and temperatures are in °C, unless specified otherwise. Experimental work is carried out at room temperature (20-25°C), unless otherwise specified. Concentrations are expressed either in parts per million ("ppm") calculated on a weight/weight basis, or on a weight/volume basis (mg/L); preferably on a weight/volume basis. The term "petroleum hydrocarbon" refers to products having a predominantly hydrocarbon composition, although they may contain minor amounts of oxygen, nitrogen, sulfur or phosphorus; petroleum hydrocarbons include crude oils as well as products derived from petroleum refining processes; they include, for example, crude oil, lubricating oil, hydraulic fluid, brake fluid, gasoline, diesel fuel, kerosene, jet fuel and heating oil. Marker compounds of this invention can be added to a petroleum hydrocarbon or a liquid biologically derived fuel; examples of the latter are biodiesel fuel, ethanol, butanol, ethyl tert-butyl ether or mixtures thereof. A substance is considered a liquid if it is in the liquid state at 20°C. A biodiesel fuel is a biologically derived fuel containing a mixture of fatty acid alkyl esters, especially methyl esters. Biodiesel fuel typically is produced by transesterification of either virgin or recycled vegetable oils, although animal fats may also be used. An ethanol fuel is any fuel containing ethanol, in pure form, or mixed with petroleum hydrocarbons, e.g., "gasohol." An "alkyl" group is a substituted or unsubstituted hydrocarbyl group having from one to twenty-two carbon atoms in a linear, branched or cyclic arrangement. Substitution on alkyl groups of one or more OH or alkoxy groups is permitted; other groups may be permitted when specified elsewhere herein. Preferably, alkyl groups are saturated. Preferably, alkyl groups are unsubstituted. Preferably, alkyl groups are linear or branched, i.e., acyclic. Preferably, each alkyl substituent is not a mixture of different alkyl groups, i.e., it comprises at least 98% of one particular alkyl group. A "heteroalkyl" group is an alkyl group in which one or more methylene groups has been replaced by O or S. Preferably, heteroalkyl groups contain from one to six O or S atoms, preferably from one to four, preferably from one to three. The methylene groups replaced by O or S were bonded to two other carbon atoms in the corresponding alkyl group. Preferably, heteroalkyl groups do not contain S atoms. Preferably, heteroalkyl groups are saturated. Heteroalkyl groups may be substituted by OH or C₁-C₁₈ alkoxy groups, preferably OH or C₁-C₆ alkoxy groups, preferably hydroxy or C₁-C₄ alkoxy groups. Examples of heteroalkyl groups include oligomers of ethylene oxide, propylene oxide or butylene oxide having two to six units of the alkylene oxide (preferably two to four, preferably two or three) and a terminal hydroxy or C₁-C₆ alkoxy group (preferably hydroxy or C₁-C₄ alkoxy, preferably hydroxy or methoxy, preferably hydroxy); an example of an ethylene oxide oligomer is -{(CH₂)₂O}ₓR, where x is an integer from two to six and R is hydrogen or C₁-C₆ alkyl. Preferably, j is from two to four, preferably two or three. Preferably, R is hydrogen or C₁-C₄ alkyl, preferably hydrogen or methyl, preferably hydrogen. Preferably, the compounds of this invention contain elements in their naturally occurring isotopic proportions.

The compounds used in the present invention are defined by the formula C{Ph(R¹)ᵢ(OR²)ⱼ}₂{Ph(R³)ₘ(OR⁴)ₙ}{Ph(R⁵)ₒ(OR⁶)ₚ}, wherein j is one or two.

Therefore, the claimed subject matters exclude compounds in which the only substitution on the four benzene rings in the formula occurs on one ring. Compounds excluded from the scope of protection include those having the structure 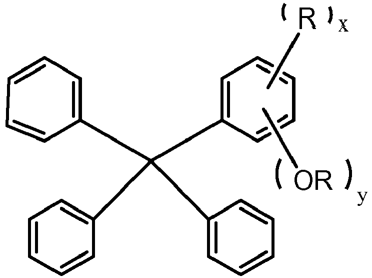 where "R" indicates any of the alkyl substituents defined herein and x and y can be zero, one or two. The compounds defined herein could not have such a structure if j is one or two because there are two benzene rings having substituents with this subscript, so if j is one or two, there would be at least one substituent on two or more rings.

Preferably, at least one of R¹, R², R³, R⁴, R⁵ and R⁶ has at least three carbon atoms, preferably at least four carbon atoms, preferably at least five. Preferably, all of the "R" groups on the compound collectively have at least six carbon atoms, preferably at least eight, preferably at least ten, preferably at least twelve; preferably no more than thirty-five, preferably no more than thirty, preferably no more than twenty-five. In one preferred embodiment, j is two. Preferably, R¹, R³ and R⁵ independently are C₁-C₁₆ alkyl or C₄-C₁₆ heteroalkyl, preferably C₁-C₁₆ alkyl, preferably C₁-C₁₂ alkyl, preferably C₃-C₁₂ alkyl, preferably C₃-C₈ alkyl, preferably C₄-C₁₂ alkyl. Preferably, R¹, R³ and R⁵ are saturated. Preferably, R¹, R³ and R⁵ are linear or branched. Preferably, R², R⁴ and R⁶ independently are C₁-C₁₆ alkyl or C₄-C₁₆ heteroalkyl, preferably C₁-C₁₆ alkyl, preferably C₁-C₁₂ alkyl, preferably C₃-C₁₂ alkyl, preferably C₃-C₈ alkyl, preferably C₄-C₁₂ alkyl. Preferably, R², R⁴ and R⁶ are saturated. Preferably, R², R⁴ and R⁶ are linear or branched.

The compounds of this invention can be depicted as follows:

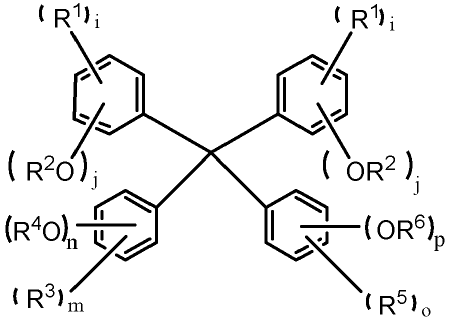

Preferably, when j is one and i, m, n, o and p are zero, then R² is not C₉-C₁₁ alkyl. This excludes compounds in which the only substituents are two C₉-C₁₁ alkoxy groups, one on each of two benzene rings. The excluded compounds would have the structure 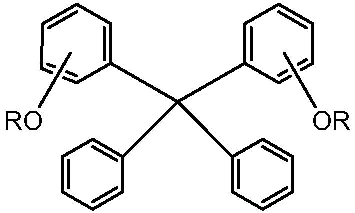 where in this case "R" indicates a C₉-C₁₁ alkyl group. Preferably, these provisos exclude C₈-C₁₂ alkyl groups.

In using the compounds described herein as markers, preferably the minimum amount of each compound added to a liquid to be marked is at least 0.01 ppm, preferably at least 0.02 ppm, preferably at least 0.05 ppm, preferably at least 0.1 ppm, preferably at least 0.2 ppm. Preferably, the maximum amount of each marker is 50 ppm, preferably 20 ppm, preferably 15 ppm, preferably 10 ppm, preferably 5 ppm, preferably 2 ppm, preferably 1 ppm, preferably 0.5 ppm. Preferably, the maximum total amount of marker compounds is 100 ppm, preferably 70 ppm, preferably 50 ppm, preferably 30 ppm, preferably 20 ppm, preferably 15 ppm, preferably 12 ppm, preferably 10 ppm, preferably 8 ppm, preferably 6 ppm, preferably 4 ppm, preferably 3 ppm, preferably 2 ppm, preferably 1 ppm. Preferably, a marker compound is not detectible by visual means in the marked petroleum hydrocarbon or liquid biologically derived fuel, i.e., it is not possible to determine by unaided visual observation of color or other characteristics that it contains a marker compound. Preferably, a marker compound is one that does not occur normally in the petroleum hydrocarbon or liquid biologically derived fuel to which it is added, either as a constituent of the petroleum hydrocarbon or liquid biologically derived fuel itself, or as an additive used therein.

Preferably, the marker compounds have a log P value of at least 3, where P is the 1-octanol/water partition coefficient. Preferably, the marker compounds have a log P of at least 4, preferably at least 5. Log P values which have not been experimentally determined and reported in the literature can be estimated using the method disclosed in Meylan, W.M & Howard, P.H., J. Pharm. Sci., vol. 84, pp. 83-92 (1995). Preferably the petroleum hydrocarbon or liquid biologically derived fuel is a petroleum hydrocarbon, biodiesel fuel or ethanol fuel; preferably a petroleum hydrocarbon or biodiesel fuel; preferably a petroleum hydrocarbon; preferably crude oil, gasoline, diesel fuel, kerosene, jet fuel or heating oil; preferably gasoline.

Preferably, the marker compounds are detected by at least partially separating them from constituents of the petroleum hydrocarbon or liquid biologically derived fuel using a chromatographic technique, e.g., gas chromatography, liquid chromatography, thin-layer chromatography, paper chromatography, adsorption chromatography, affinity chromatography, capillary electrophoresis, ion exchange and molecular exclusion chromatography. Chromatography is followed by at least one of: (i) mass spectral analysis, and (ii) FTIR. Identities of the marker compounds preferably are determined by mass spectral analysis. Preferably, mass spectral analysis is used to detect the marker compounds in the petroleum hydrocarbon or liquid biologically derived fuel without performing any separation. Alternatively, marker compounds may be concentrated prior to analysis, e.g., by distilling some of the more volatile components of a petroleum hydrocarbon or liquid biologically derived fuel.

Preferably, more than one marker compound is present. Use of multiple marker compounds facilitates incorporation into the petroleum hydrocarbon or liquid biologically derived fuel of coded information that may be used to identify the origin and other characteristics of the petroleum hydrocarbon or liquid biologically derived fuel. The code comprises the identities and relative amounts, e.g., fixed integer ratios, of the marker compounds. One, two, three or more marker compounds may be used to form the code. Marker compounds according to this invention may be combined with markers of other types, e.g., markers detected by absorption spectrometry, including those disclosed in U.S. Pat. No. 6,811,575; U.S. Pat. App. Pub. No. 2004/0250469 and EP App. Pub. No. 1,479,749. Marker compounds are placed in the petroleum hydrocarbon or liquid biologically derived fuel directly, or alternatively, placed in an additives package containing other compounds, e.g., antiwear additives for lubricants, detergents for gasoline, etc., and the additives package is added to the petroleum hydrocarbon or liquid biologically derived fuel.

The compounds of this invention may be prepared by methods known in the art, e.g., allowing a substituted benzene (substituted with alkyl and/or hydroxyl groups) to react with a carbon tetrahalide and a Lewis acid to form a substituted dihalobenzophenone precursor, then allowing said precursor to react with another substituted benzene to form a substituted tetraphenylmethane. Hydroxyl groups on the substituted tetraphenylmethane can be converted to alkyl ethers by reaction with, e.g., an alkyl halide, in the presence of a base, e.g., alkali metal hydroxides, preferably in a polar aprotic solvent, e.g., DMSO, NMP, DMAc. Another method comprises allowing a substituted benzophenone, which may be asymmetric, to react with a halogenating agent, e.g., phosphorus pentachloride, to form a substituted dichlorodiphenylmethane, followed by reaction with a substituted or unsubstituted phenol under Friedel-Crafts conditions.

### EXAMPLES

### Syntheses of 4,4'-Dihydroxytetraphenylmethane (DHTPM) Ethers

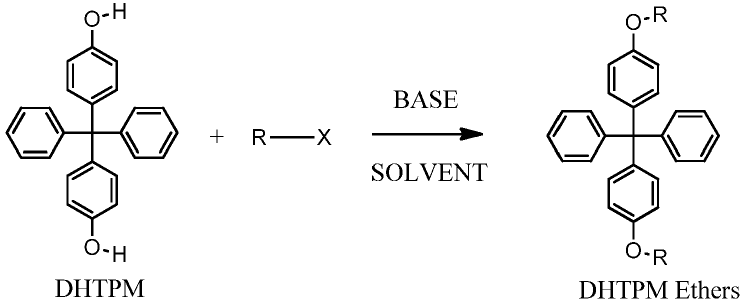

X = Cl, Br
1) Bis(4-(pentyloxy)phenyl)diphenylmethane (BPentTPM): A 125 mL 3-neck flask was equipped with a magnetic stirrer, a reflux condenser with nitrogen blanket, and a heating mantle with a temperature controller and a thermocouple. The flask was charged with 3.54 grams (0.01 moles) of 4,4'-dihydroxy-tetraphenylmethane (DHTPM), 1.35 grams (0.02 moles) of potassium hydroxide (KOH) pellets, and 40 mL of dimethylsulfoxide (DMSO). The mixture was stirred under nitrogen and was heated to 100°C. The KOH was completely dissolved by the time the temperature reached 100°C. The reaction mixture was cooled to below 50°C, and then 2.48 mL (d 1.218; 3.02 grams; 0.02 moles) of 1-bromopentane were added in one portion. An exotherm of about 8°C was noted. The reaction mixture was then heated to 70 - 75°C, the reaction being monitored by GPC analyses. Solids began to separate out from the mixture as soon as the reaction mixture temperature reached about 60°C. After 3 hours at 70 - 75°C, the reaction mixture was poured into about 250 mL of water containing about 25 grams of sodium chloride and a few pellets of KOH. Toluene (about 150 mL was added, and the mixture was stirred at room temperature for about 30 minutes. The mixture was transferred to a separatory funnel, and the layers were separated. The lower, aqueous, layer was extracted with 1 x 50 mL of toluene. The toluene layers were combined and were washed with 1 x 50 mL of saturated aqueous sodium chloride solution, and were then dried over anhydrous magnesium sulfate. The toluene mixture was filtered, and the solvent was removed by rotary evaporation to give 4.35 grams (88.2% yield) of BPentTPM as a light brown crystalline solid. MP = 110 - 113°C. The structure of the product was confirmed by IR, NMR, and GC/MS analyses.
2) Bis(4-hexyloxy)phenyl)diphenylmethane (BHexTPM): This ether was synthesized as described above from 3.52 grams (0.01 moles) of DHTPM, 1.34 grams (0.02 moles) of KOH, 25 mL of DMSO, and 2.81 mL (d 1.176; 3.30 grams; 0.02 moles) of 1-bromohexane. The reaction mixture was heated at 70 - 75°C for 3 hours after the addition of the bromide. The yield of product as a beige solid was 4.64 grams (89.1%). MP = 120 - 122°C. The structure of the product was confirmed by IR, NMR, and GC/MS analyses.
3) Bis(4-(heptyloxy)phenyl)diphenylmethane (BHeptTPM): This ether was synthesized as described above from3.53 grams (0.01 moles) of DHTPM, 1.34 grams (0.02 moles) of KOH, 40 mL of DMSO, and 3.14 mL (d 1.14; 3.58 grams; 0.02 moles) of 1-bromoheptane. The reaction mixture was heated at 70 - 75°C for 5.5 hours after the addition of the bromide. The yield of product as a beige crystalline solid was 5.06 grams (92.2%). MP = 103 - 105°C. The structure of the product was confirmed by IR, NMR, and GC/MS analyses.
4) Bis(4-(octyloxy)phenyl)diphenylmethane (BOctTPM): This ether was synthesized as described above from3.53 grams (0.01 moles) of DHTPM, 1.4 grams (0.029 moles) of KOH, 40 mL of DMSO, and 3.45 mL (d 1.118; 3.86 grams; 0.02 moles) of 1-bromooctane. The reaction mixture was heated at 70 - 75°C for 8 hours after the addition of the bromide. GPC analyses showed the presence of some remaining DHTPM. Upon work up, the amount of KOH present in the aqueous solution was increased from a few pellets to about 5 grams, and the mixture was stirred at room temperature overnight instead of for only 30 minutes. The yield of product as a beige crystalline solid was 5.29 grams (91.7%). MP = 84.5 - 86.5°C. The structure of the product was confirmed by IR, NMR, and GC/MS analyses.

### Analyses

1. IR Analyses: IR analyses were performed using a Nicolet 560 FTIR spectrometer. For liquid samples, a small drop was cast as a neat film between two KBr plates. For solid samples, KBr dispersions were pressed. The IR spectrum was acquired in the transmission mode from 4000 to 400 cm⁻¹, with a spectral resolution of 4 cm⁻¹. A Happ-Genzel type apodization function was used.
2. NMR Analyses: Both ¹H and ¹³C NMR spectra were acquired using a Bruker 200 NMR spectrometer operating at 4.7 T. ¹H spectra were obtained using an 8.2 second accumulation time and 2.0 KHz sweep width; the ¹³C spectra were obtained at a 4.7 second accumulation time and 7.0 KHz sweep width. Methanol-d₄ was typically used as the solvent. Chemical shifts were referenced using the solvent resonances at 3.30 ppm for ¹H, and at 59.05 ppm for ¹³C.
3. GPC Analyses: GPC analyses to follow the progress of synthesis reactions and to determine product purity were performed using a PerkinElmer Series 200 HPLC. Two Polymer Laboratories pLgel columns were used in series: 1) 300 mm x 7.5 mm, 3µ, 100 Ǻ and 2) 300 mm x 7.5 mm, 5µ, 50 Ǻ. These two columns were preceded by a guard column. The columns were maintained at 35°C. The mobile phase was 100% THF at a flow rate of 2 mL / minute. UV detection was at 270 nm. The program run time was 10 minutes.
4. GC / MS Analyses: These analyses gave GC retention time and MS fragmentation data, and were performed using a Hewlett Packard Model 6890 GC system with an Agilent Mass Selective Detector operating in electron ionization (EI) mode and in positive chemical ionization (CI) mode. The carrier gas for the EI mode was helium at approximately 1 mL / minute. Methane was used as the carrier gas for the CI mode. The column was a J&W Scientific DB-5MS, 30 meter x 0.25 mm x 1 µm film. The initial oven temperature was 60°C with a hold time of 5 minutes. The temperature was ramped at 10°C / minute to 220°C with a hold of 2 minutes, and then it was ramped at 20°C / minute to 290°C. The injector temperature was 225°C. The sample size was 1 µL for EI mode, and 1 µL for CI mode. The split ratio was 50:1.
5. Melting Points: Melting points were determined using a Mel-Temp apparatus, and are uncorrected.

### Detection and Linearity Study for BHexTPM

Instrument: 6890 GC with 5973 MSD, and 7683B autosampler
Injection port: 280°C, 3 µL injection, splitless
Column: DB-35, 15m, 0.255 mm ID, 0.25 µm film
Flow: 1.4 mL/min He
Oven: 100°C, hold 0 min, ramp 20°C /min to 280°C, hold 10 min, ramp 20°C /min to 340°C, hold 5 min
Aux transfer line: 280°C
Solvent delay: 17 min
Mass detection mode: SIM mode, for ions 343.3, 443.4, 520.4 (0.7-0.9 amu)
Dwell time: 100 msec
MS Source: EI, 250°C
MS Quad: 200°C

### Sample dilution:

Stock 1: 10 mg BHexTPM dissolved in 25 mL xylene
Stock 2: 0.5 mL Stock 1 diluted to 25 mL in xylene
1000ppb: 1.25 mL Stock 2 diluted to 10 mL in Turkish diesel
500ppb: 625 µL Stock 2 diluted to 10 mL in Turkish diesel
250ppb: 312.5 µL Stock 2 diluted to 10 mL in Turkish diesel
100ppb: 1 mL of 1000ppb stock diluted to 10 mL in Turkish diesel
50ppb: 1 mL of 500ppb stock diluted to 10 mL in Turkish diesel

| **ppb** | **Ret Time** | **Width** | **Area** | **Start Time** | **End Time** |
|---|---|---|---|---|---|
| 1000 | 23.000 | 0.066 | 9887889 | 22.911 | 23.192 |
| 500 | 23.000 | 0.071 | 5125725 | 22.907 | 23.298 |
| 250 | 23.001 | 0.072 | 2493155 | 22.907 | 23.274 |
| 100 | 23.000 | 0.074 | 1039475 | 22.854 | 23.298 |
| 50 | 22.998 | 0.07 | 507502 | 22.854 | 23.274 |

Results of linear regression analysis: Area = 9888.8 X concentration + 53010; R² = 0.9996

| STRUCTURE | MP, °C | GC RET. TIME (min) | MS Masses | Solubility @ 10% @ -10C | GPC RET TIME (MIN) |
|---|---|---|---|---|---|
| 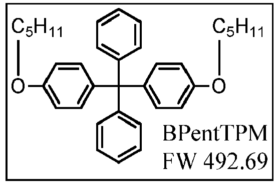 | 110 - 113 | 21.9 | 492, 415, 329 | | 6.8 |
| 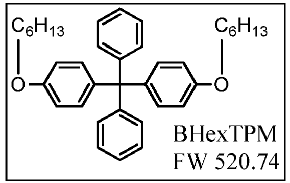 | 120 - 122 | 23.1 | 520, 443, 343 | | 6.68 |
| 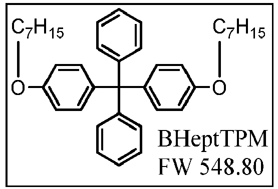 | 103 - 105 | 24.4 | 548, 471, 357 | | 6.61 |
| 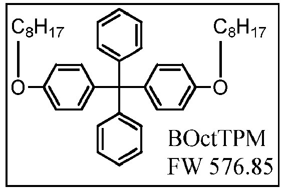 | 84.5 - 86.5 | 26.2 | 576, 499, 371 | | 6.57 |

## Claims

1. A method for marking a petroleum hydrocarbon or a liquid biologically derived fuel; said method comprising adding to said petroleum hydrocarbon or liquid biologically derived fuel at least one C{Ph(R¹)ᵢ(OR²)ⱼ}₂{Ph(R³)ₘ(OR⁴)ₙ}{Ph(R⁵)ₒ(OR⁶)ₚ}, wherein Ph represents a benzene ring; R¹, R³ and R⁵ independently are C₁-C₁₈ alkyl or C₄-C₁₈ heteroalkyl; R², R⁴ and R⁶ independently are C₁-C₁₈ alkyl or C₄-C₁₈ heteroalkyl; i, m, n, o and p independently are zero, one or two; and j is one or two; wherein each compound having formula C{Ph(R¹)ᵢ(OR²)ⱼ}₂{Ph(R³)ₘ(OR⁴)ₙ}{Ph(R⁵)ₒ(OR⁶)ₚ} is present at a level from 0.01 ppm to 20 ppm.

2. The method of claim 1 in which all R¹, R², R³, R⁴, R⁵ and R⁶ substituents collectively have a total of eight to thirty-five carbon atoms.

3. The method of claim 2 in which at least one of R¹, R², R³, R⁴, R⁵ and R⁶ has at least three carbon atoms.

4. The method of claim 3 in which R¹, R², R³, R⁴, R⁵ and R⁶ are independently are C₁-C₁₆ alkyl.

5. The method of claim 4 in which each compound having formula C{Ph(R¹)ᵢ(OR²)ⱼ}₂{Ph(R³)ₘ(OR⁴)ₙ}{Ph(R⁵)ₒ(OR⁶)ₚ} is present at a level from 0.01 ppm to 10 ppm.

6. The method of claim 5 in which R¹, R², R³, R⁴, R⁵ and R⁶ are saturated and acyclic.

7. The method of claim 1 in which at least one of R¹, R², R³, R⁴, R⁵ and R⁶ has at least three carbon atoms.

8. The method of claim 7 in which R¹, R², R³, R⁴, R⁵ and R⁶ are independently are C₁-C₁₆ alkyl.

9. The method of claim 8 in which each compound having formula C{Ph(R¹)ᵢ(OR²)ⱼ}₂{Ph(R³)ₘ(OR⁴)ₙ}{Ph(R⁵)ₒ(OR⁶)ₚ} is present at a level from 0.01 ppm to 10 ppm.

## Patentansprüche

1. Ein Verfahren zum Markieren eines Erdölkohlenwasserstoffs oder eines flüssigen biologisch abgeleiteten Brennstoffs; wobei das Verfahren das Zugeben zu dem Erdölkohlenwasserstoff oder dem flüssigen biologisch abgeleiteten Brennstoff von mindestens einem C{Ph(R¹)ᵢOR²)ⱼ}₂{Ph(R³)ₘ(OR⁴)ₙ}{Ph(R⁵)ₒ(OR⁶)ₚ} beinhaltet, wobei Ph einen Benzolring darstellt; R¹, R³ und R⁵ unabhängig C₁-C₁₈-Alkyl oder C₄-C₁₈-Heteroalkyl sind; R², R⁴ und R⁶ unabhängig C₁-C₁₈-Alkyl oder C₄-C₁₈-Heteroalkyl sind; i, m, n, o und p unabhängig null, eins oder zwei sind; und j eins oder zwei ist; wobei jede Verbindung, die Formel C{Ph(R¹)ⱼ(OR²)ⱼ}₂{Ph(R³)ₘ(OR⁴)ₙ}{Ph(R⁵)ₒ(OR⁶)ₚ} aufweist, in einer Menge von 0,01 ppm bis 20 ppm vorliegt.

2. Verfahren gemäß Anspruch 1, wobei alle Substituenten R¹, R², R³, R⁴, R⁵ und R⁶ zusammen insgesamt acht bis fünfunddreißig Kohlenstoffatome aufweisen.

3. Verfahren gemäß Anspruch 2, wobei mindestens eines von R¹, R², R³, R⁴, R⁵ und R⁶ mindestens drei Kohlenstoffatome aufweist.

4. Verfahren gemäß Anspruch 3, wobei R¹, R², R³, R⁴, R⁵ und R⁶ unabhängig C₁-C₁₆-Alkyl sind.

5. Verfahren gemäß Anspruch 4, wobei jede Verbindung, die Formel C{Ph(R¹)ᵢ(OR²)ⱼ}₂{Ph(R³)ₘ(OR⁴)ₙ}{Ph(R⁵)ₒ(OR⁶)ₚ} aufweist, in einer Menge von 0,01 ppm bis 10 ppm vorliegt.

6. Verfahren gemäß Anspruch 5, wobei R¹, R², R³, R⁴, R⁵ und R⁶ gesättigt und acyclisch sind.

7. Verfahren gemäß Anspruch 1, wobei mindestens eines von R¹, R², R³, R⁴, R⁵ und R⁶ mindestens drei Kohlenstoffatome aufweist.

8. Verfahren gemäß Anspruch 7, wobei R¹, R², R³, R⁴, R⁵ und R⁶ unabhängig C₁-C₁₆-Alkyl sind.

9. Verfahren gemäß Anspruch 8, wobei jede Verbindung, die Formel C{Ph(R¹)ᵢOR²)ⱼ}₂{Ph(R³)ₘ(OR⁴)ₙ}{Ph(R⁵)ₒ(OR⁶)ₚ} aufweist, in einer Menge von 0,01 ppm bis 10 ppm vorliegt.

## Revendications

1. Un procédé pour marquer un hydrocarbure de pétrole ou un combustible liquide d'origine biologique ; ledit procédé comprenant l'ajout audit hydrocarbure de pétrole ou combustible liquide d'origine biologique d'au moins un C{Ph(R¹)ᵢ(OR²)ⱼ}₂{Ph(R³)ₘ(OR⁴)ₙ}{Ph(R⁵)ₒ(OR⁶)ₚ}, où Ph représente un noyau benzénique ; R¹, R³ et R⁵ sont indépendamment un alkyle en C₁-C₁₈ ou un hétéroalkyle en C₄-C₁₈ ; R², R⁴ et R⁶ sont indépendamment un alkyle en C₁-C₁₈ ou un hétéroalkyle en C₄-C₁₈ ; i, m, n, o et p sont indépendamment zéro, un ou deux ; et j est un ou deux ; où chaque composé ayant la formule C{Ph(R¹)ᵢ(OR²)ⱼ}₂{Ph(R³)ₘ(OR⁴)ₙ}{Ph(R⁵)(OR⁶)ₚ} est présent à un taux allant de 0,01 ppm à 20 ppm.

2. Le procédé de la revendication 1 dans lequel tous les substituants R¹, R², R³, R⁴, R⁵ et R⁶ ont collectivement un total de huit à trente-cinq atomes de carbone.

3. Le procédé de la revendication 2 dans lequel au moins un R parmi R¹, R², R³, R⁴, R⁵ et R⁶ a au moins trois atomes de carbone.

4. Le procédé de la revendication 3 dans lequel R¹, R², R³, R⁴, R⁵ et R⁶ sont indépendamment un alkyle en C₁-C₁₆.

5. Le procédé de la revendication 4 dans lequel chaque composé ayant la formule C{Ph(R¹)ᵢ(OR²)ⱼ}₂{Ph(R³)ₘ(OR⁴)ₙ}{Ph(R⁵)(OR⁶)ₚ} est présent à un taux allant de 0,01 ppm à 10 ppm.

6. Le procédé de la revendication 5 dans lequel R¹, R², R³, R⁴, R⁵ et R⁶ sont saturés et acycliques.

7. Le procédé de la revendication 1 dans lequel au moins un R parmi R¹, R², R³, R⁴, R⁵ et R⁶ a au moins trois atomes de carbone.

8. Le procédé de la revendication 7 dans lequel R¹, R², R³, R⁴, R⁵ et R⁶ sont indépendamment un alkyle en C₁-C₁₆.

9. Le procédé de la revendication 8 dans lequel chaque composé ayant la formule C{Ph(R¹)ᵢ(OR²)ⱼ}₂{Ph(R³)ₘ(OR⁴)ₙ}{Ph(R⁵)ₒ(OR⁶)ₚ} est présent à un taux allant de 0,01 ppm à 10 ppm.
